# EUROPEAN PATENT APPLICATION

(11) **EP 4 119 194 A1**
(43) Date of publication of application: **18.01.2023**
(21) Application number: 21767241.9
(22) Date of filing: 10.03.2021
(51) Int. Cl.: A61Q 1/00, A61K 8/25, A61K 8/31, A61K 8/37, A61K 8/87, A61K 8/89

(54) **OILY COMPOSITION AND COSMETICS COMPOSITION CONTAINING SAID OILY COMPOSITION**

(30) Priority: 13.03.2020 JP 2020043860
(71) Applicant: ADEKA CORPORATION, Arakawa-ku Tokyo 116-8554 (JP)
(72) Inventor: MIYAZONO, Keitarou, Tokyo 116-8554 (JP); MAEBASHI, Marika, Tokyo 116-8554 (JP); YASUTANI, Akihito, Tokyo 116-8554 (JP); MATSUKURA, Noriyoshi, Tokyo 116-8554 (JP); TSUSHIMA, Yasuhiro, Tokyo 116-8554 (JP); SHIRAI, Hiroaki, Tokyo 116-8554 (JP)
(74) Representative: Müller-Boré & Partner Patentanwälte PartG mbB
(86) International application number: PCT/JP2021/009479
(87) International publication number: WO 2021/182500

(57) **Abstract**

The present invention provides an oily composition, including: at least one kind of urethane polymer (A) selected from the group consisting of a (PPG-12/SMDI) copolymer and a (polyglyceryl-2 diisostearate/IPDI) copolymer; silica particles (B); and one or more kinds of oil components (C) selected from an ester oil, a hydrocarbon oil, and a silicone oil, and a cosmetic composition including the oily composition.

## Description

### Technical Field

The present invention relates to an oily composition showing a satisfactory touch feeling and a satisfactory thickening effect, and a cosmetic composition showing an excellent use feeling and an excellent thickening effect.

### Background Art

A product, such as a coating material, a cleaning agent, an adhesive, a cosmetic, a drug, an agricultural chemical, an ink, or a dye, is in the form of an aqueous or oily composition, and various thickeners or viscosity-adjusting agents have been used for obtaining a composition having viscosity or fluidity suitable for use. In, for example, Patent Document 1, there is a description of a method by which extremely effective polyurethane suitable for a thickener for an aqueous system is easily produced. In addition, in Patent Document 2, as a viscosity-adjusting agent that shows small temperature dependence and hence keeps a certain range of viscosity under any conditions, there is a description of a viscosity-adjusting agent consisting of a specific urethane compound. In addition, the development of, in particular, a thickener or a viscosity-adjusting agent for modifying the viscosity or fluidity of an oily composition has been vigorously performed, and in, for example, Patent Document 3, there is a description of a thickener for an oil that can thicken an oil, such as an oil and fat, a mineral oil, or a synthetic oil, into a gel-like product or a jelly-like product having an elastic feeling.

In addition, various thickeners or viscosity-adjusting agents except such urethane polymer-type products as described above have been developed. For example, in Patent Document 4, there is a description of a viscosity-adjusting agent for an aqueous coating material consisting of a smectite-based clay mineral and a condensed phosphate, and in Patent Document 5, there is a description of a thickener for a liquid consisting of silanized and pyrolytically produced silicic acid.

However, each of the above-mentioned related-art viscosity-adjusting agents has involved a problem in that its addition amount needed for imparting, to various compositions, viscosity or fluidity suitable for use becomes larger to result in the impairment of the touch feelings of the compositions such as the occurrence of stickiness. Accordingly, there has been required a material, which easily adjusts the viscosity of a product and can satisfactorily improve the viscosity without causing deterioration of the use feeling of the product such as stickiness.

### Citation List

### Patent Documents

[Patent Document 1] JP H8-253548 A
[Patent Document 2] JP H9-71767 A
[Patent Document 3] JP 2011-102256 A
[Patent Document 4] JP H6-299104 A
[Patent Document 5] JP H7-232912 A

### Summary of Invention

### Technical Problem

Accordingly, an object of the present invention is to provide an oily composition, which easily adjusts the viscosity of a product, and imparts a satisfactory touch feeling and a satisfactory thickening effect to the product, and a cosmetic composition, which comprises the oily composition and shows an excellent use feeling.

### Solution to Problem

In view of the foregoing, the inventors of the present invention made extensive investigations, and as a result, found an oily composition showing a satisfactory touch feeling and a satisfactory thickening effect. Thus, the inventors reached the present invention. That is, according to one embodiment of the present invention, there is provided an oily composition, comprising: at least one kind of urethane polymer (A) selected from the group consisting of a (PPG-12/SMDI) copolymer and a (polyglyceryl-2 diisostearate/IPDI) copolymer; silica particles (B); and one or more kinds of oil components (C) selected from an ester oil, a hydrocarbon oil, and a silicone oil.

### Advantageous Effects of Invention

The oily composition of the present invention is an oily composition that imparts an excellent thickening effect to a product, and when the composition is used in a product, such as a coating material, a cleaning agent, an adhesive, a cosmetic, a drug, an agricultural chemical, an ink, or a dye, the composition can easily adjust the viscosity of the product to one suitable for each purpose. In particular, when the oily composition of the present invention is used as a material for a product to be applied to skin such as a cosmetic preparation, the product shows an excellent touch feeling, an excellent use feeling, and an excellent thickening effect.

### Description of Embodiments

A urethane polymer (A) to be used in the present invention is at least one kind of urethane polymer selected from the group consisting of a (PPG-12/SMDI) copolymer and a (polyglyceryl-2 diisostearate/IPDI) copolymer. Each of the (PPG-12/SMDI) copolymer and the (polyglyceryl-2 diisostearate/IPDI) copolymer is a urethane polymer showing solubility in various oil components. In the present invention, when the at least one kind of urethane polymer (A) selected from the group consisting of the (PPG-12/SMDI) copolymer and the (polyglyceryl-2 diisostearate/IPDI) copolymer is used, there can be obtained an oily composition, which shows a satisfactory touch feeling and a satisfactory thickening effect at the time of the combination of the polymer with an oil component (C) through a synergistic thickening effect with silica particles (B).

The (PPG-12/SMDI) copolymer is a copolymer of PPG-12 (polypropylene glycol: average polymerization number of propylene glycol=12) and dicyclohexylmethane 4,4'-diisocyanate (SMDI). Although the molecular weight of the (PPG-12/SMDI) copolymer to be used in the present invention is not particularly limited, the weight-average molecular weight of the (PPG-12/SMDI) copolymer is preferably from 1,000 to 500,000, more preferably from 1,500 to 100,000, still more preferably from 1,500 to 50,000, still more preferably from 2,000 to 10,000 from the viewpoints of a thickening effect by the oily composition to be obtained, and the touch feeling and use feeling of a cosmetic composition comprising the oily composition. In the present invention, the weight-average molecular weight of the (PPG-12/SMDI) copolymer may be determined in terms of styrene through measurement by gel permeation chromatography (GPC).

Measurement conditions for the GPC are as described below.
[GPC Measurement Conditions]
Apparatus: LC-2000Plus series (manufactured by JASCO Corporation)
Column: KF-803 (manufactured by Showa Denko K.K.)
Column temperature: 40°C
Sample concentration: 0.1 mass%
Developing solvent: Tetrahydrofuran
Detector: An intelligent differential refractometer RI-2031 (manufactured by JASCO Corporation)
Reference substance: TSKgel standard polystyrene (manufactured by Tosoh Corporation)

The (polyglyceryl-2 diisostearate/IPDI) copolymer is a copolymer of polyglyceryl-2 diisostearate and isophorone diisocyanate (IPDI). Although the molecular weight of the (polyglyceryl-2 diisostearate/IPDI) copolymer to be used in the present invention is not particularly limited, the weight-average molecular weight of the (polyglyceryl-2 diisostearate/IPDI) copolymer is preferably from 1,000 to 500,000, more preferably from 1,500 to 100,000, still more preferably from 1,500 to 50,000, still more preferably from 2,000 to 10,000 from the viewpoints of the thickening effect by the oily composition to be obtained, and the touch feeling and use feeling of the cosmetic composition. In the present invention, the weight-average molecular weight of the (polyglyceryl-2 diisostearate/IPDI) copolymer may be determined in terms of styrene through measurement by gel permeation chromatography (GPC).

The urethane polymer (A) to be used in the present invention may be any one of the following types: the polymer consists of the (PPG-12/SMDI) copolymer; the polymer consists of the (polyglyceryl-2 diisostearate/IPDI) copolymer; and the polymer is a mixture consisting of the (PPG-12/SMDI) copolymer and the (polyglyceryl-2 diisostearate/IPDI) copolymer. When the urethane polymer (A) is a mixture consisting of the (PPG-12/SMDI) copolymer and the (polyglyceryl-2 diisostearate/IPDI) copolymer, a content ratio between the (PPG-12/SMDI) copolymer and the (polyglyceryl-2 diisostearate/IPDI) copolymer is not particularly limited, but may be, for example, from 5:95 to 95:5 in terms of mass ratio.

A commercial product may be used as each of the (PPG-12/SMDI) copolymer and the (polyglyceryl-2 diisostearate/IPDI) copolymer to be used in the present invention, or each of the copolymers may be produced by a known production method. Examples of a method of producing the (PPG-12/SMDI) copolymer include: a method involving causing PPG-12 and SMDI to react with each other until no reactive isocyanate group is present; and a method involving causing PPG-12 and SMDI to react with each other to produce a urethane prepolymer, and then causing the prepolymer to react with a chain extender. In addition, examples of a method of producing the (polyglyceryl-2 diisostearate/IPDI) copolymer include: a method involving causing polyglyceryl-2 diisostearate and IPDI to react with each other until no reactive isocyanate group is present; and a method involving causing polyglyceryl-2 diisostearate and IPDI to react with each other to produce a urethane prepolymer, and then causing the prepolymer to react with a chain extender. In addition, although the kind of the chain extender when the chain extender is used in each of the production methods is not particularly limited, for example, one or more kinds selected from the group consisting of water, ethylenediamine, and propylenediamine may be used.

Although the usage amount of each of the urethane prepolymer produced by causing PPG-12 and SMDI to react with each other, and the chain extender at the time of the reaction therebetween is not particularly limited, the ratio of the number of moles of the chain extender to the number of moles of isocyanate groups remaining in the urethane prepolymer (number obtained by subtracting the number of moles of the hydroxy group of PPG-12 from the number of moles of the isocyanate groups of SMDI used) is preferably 1.0 times or more (in other words, the number of moles of the chain extender is preferably equal to or more than the number of moles of the isocyanate groups remaining in the urethane prepolymer) from the viewpoints of the various characteristics of the (PPG-12/SMDI) copolymer to be obtained. In the present invention, the extension of the molecular chain of the urethane prepolymer with the chain extender can easily adjust the molecular weight of the (PPG-12/SMDI) copolymer to be obtained within a desired range. In addition, when water is used as the chain extender, the water may be used as a solvent or a dispersion medium for the (PPG-12/SMDI) copolymer to be obtained. Accordingly, the adjustment of the usage amount of the water can provide aqueous solutions or dispersion liquids of the (PPG-12/SMDI) copolymer having various concentrations.

Although the usage amount of each of the urethane prepolymer produced by causing polyglyceryl-2 diisostearate and IPDI to react with each other, and the chain extender at the time of the reaction therebetween is not particularly limited, the ratio of the number of moles of the chain extender to the number of moles of isocyanate groups remaining in the urethane prepolymer (number obtained by subtracting the number of moles of the hydroxy group of polyglyceryl-2 diisostearate from the number of moles of the isocyanate groups of IPDI used) is preferably 1.0 times or more (in other words, the number of moles of the chain extender is preferably equal to or more than the number of moles of the isocyanate groups remaining in the urethane prepolymer) from the viewpoints of the various characteristics of the (polyglyceryl-2 diisostearate/IPDI) copolymer to be obtained. In the present invention, the extension of the molecular chain of the urethane prepolymer with the chain extender can easily adjust the molecular weight of the (polyglyceryl-2 diisostearate/IPDI) copolymer to be obtained within a desired range. In addition, when water is used as the chain extender, the water may be used as a solvent or a dispersion medium for the (polyglyceryl-2 diisostearate/IPDI) copolymer to be obtained. Accordingly, the adjustment of the usage amount of the water can provide aqueous solutions or dispersion liquids of the (polyglyceryl-2 diisostearate/IPDI) copolymer having various concentrations.

In addition, methods of producing the urethane prepolymers from the respective raw materials are not particularly limited, and the prepolymers may each be produced by a known method. There may be used, for example, a prepolymer mixing method involving: causing polyglyceryl-2 diisostearate and IPDI, or PPG-12 and SMDI, to react with each other; and adding the resultant to a solvent containing an emulsifying agent to disperse the resultant therein as required.

Examples of the emulsifying agent that may be used in each of the methods of producing the (PPG-12/SMDI) copolymer and the (polyglyceryl-2 diisostearate/IPDI) copolymer include: a known anionic surfactant, a known non-ionic surfactant, and a known cationic surfactant; and a mixture of these surfactants. Examples of the solvent include ethanol, propanol, butanol, hexane, toluene, ethyl acetate, butyl acetate, and water.

Although a usage ratio between PPG-12 and SMDI to be used in the production of the (PPG-12/SMDI) copolymer is not particularly limited, PPG-12 and SMDI are preferably used at a molar ratio of from 1:5 to 20:1, are more preferably used at a molar ratio of from 1:1 to 10:1, and are still more preferably used at a molar ratio of from 2:1 to 5:1 from the viewpoints of the various characteristics of the (PPG-12/SMDI) copolymer to be obtained.

In the method of producing the (PPG-12/SMDI) copolymer, any other raw material that can react with PPG-12 or SMDI may be used to the extent that the effects of the present invention are not impaired. However, any one of the following production methods is preferably adopted from the viewpoints of the effects of the present invention: a method in which raw materials consisting of PPG-12 and SMDI are used, and are caused to react with each other until no reactive isocyanate group is present; and a method involving causing only the chain extender to react with the urethane prepolymer obtained by causing the raw materials consisting of PPG-12 and SMDI to react with each other.

In the present invention, the following method is particularly preferably used as the method of producing the (PPG-12/SMDI) copolymer from the viewpoint of particularly improving the thickening effect of the oily composition to be obtained and the touch feeling of the cosmetic composition through a synergistic thickening effect of the urethane polymer (A) and the silica particles (B): a method in which a molar ratio between PPG-12 and SMDI in the raw materials consisting of PPG-12 and SMDI is from 2:1 to 5:1, and the raw materials are caused to react with each other until no reactive isocyanate group is present.

Although a usage ratio between polyglyceryl-2 diisostearate and IPDI to be used in the method of producing the (polyglyceryl-2 diisostearate/IPDI) copolymer is not particularly limited, polyglyceryl-2 diisostearate and IPDI are preferably used at a molar ratio of from 1:5 to 20:1, are more preferably used at a molar ratio of from 1:1 to 10:1, and are still more preferably used at a molar ratio of from 2:1 to 5:1 from the viewpoints of the various characteristics of the (polyglyceryl-2 diisostearate/IPDI) copolymer to be obtained.

In the method of producing the (polyglyceryl-2 diisostearate/IPDI) copolymer, any other raw material that can react with polyglyceryl-2 diisostearate or IPDI may be used to the extent that the effects of the present invention are not impaired. However, any one of the following production methods is preferably adopted from the viewpoints of the effects of the present invention: a method in which raw materials consisting of polyglyceryl-2 diisostearate and IPDI are used, and are caused to react with each other until no reactive isocyanate group is present; and a method involving causing only the chain extender to react with the urethane prepolymer obtained by causing the raw materials consisting of polyglyceryl-2 diisostearate and IPDI to react with each other.

In the present invention, the following method is particularly preferably used as the method of producing the (polyglyceryl-2 diisostearate/IPDI) copolymer from the viewpoint of particularly improving the thickening effect of the oily composition to be obtained and the touch feeling of the cosmetic composition through a synergistic thickening effect of the urethane polymer (A) and the silica particles (B): a method in which a molar ratio between polyglyceryl-2 diisostearate and IPDI in the raw materials consisting of polyglyceryl-2 diisostearate and IPDI is from 2:1 to 5:1, and the raw materials are caused to react with each other until no reactive isocyanate group is present.

Each of the reactions of the methods of producing the (PPG-12/SMDI) copolymer and the (polyglyceryl-2 diisostearate/IPDI) copolymer is not particularly limited as long as the reaction is performed under such a condition that the respective raw materials can react with each other. In addition, the respective raw materials may be caused to react with each other after their total amount has been collectively loaded, or the raw materials may be caused to react with each other while being loaded in several portions. The following method is given as a specific example: the respective raw material components are loaded into a reaction system in one stroke or in several portions, and are mixed at a temperature of from 30°C to 160°C, preferably from 40°C to 160°C under a pressurized, reduced-pressure, or normal-pressure environment, followed by the maintenance of the mixture for from 30 minutes to 10 hours until a reaction between the raw materials is completed.

In addition, in each of the reactions of the methods of producing the (PPG-12/SMDI) copolymer and the (polyglyceryl-2 diisostearate/IPDI) copolymer, a catalyst may be used for accelerating the reaction. Examples of the catalyst include: strong acids, such as sulfuric acid and toluenesulfonic acid; metal halides, such as titanium tetrachloride, hafnium chloride, zirconium chloride, aluminum chloride, gallium chloride, indium chloride, iron chloride, tin chloride, and boron fluoride; hydroxides, alcoholates, and carbonates of alkali metals and alkaline earth metals, such as sodium hydroxide, potassium hydroxide, sodium methylate, and sodium carbonate; metal oxides, such as aluminum oxide, calcium oxide, barium oxide, and sodium oxide; organic metal compounds, such as tetraisopropyl titanate, dibutyltin dichloride, dibutyltin oxide, and dibutyltin bis(2-ethylhexylthioglycolate); and soaps, such as sodium acetate, potassium acetate, sodium propionate, potassium propionate, sodium octylate, potassium octylate, sodium laurate, and potassium laurate. Although the blending amount of such catalyst is not particularly limited, the amount is from 0.01 mass% to 1 mass% with respect to the total mass of the respective raw materials. Although the reaction advances even when no catalyst is used, the use of the catalyst increases the rate of the reaction, and hence provides a reaction time-shortening effect.

Although the content of the urethane polymer (A) in the oily composition of the present invention is not particularly limited, the content of the urethane polymer (A) in the oily composition of the present invention is preferably from 0.1 mass% to 25 mass%, more preferably from 0.2 mass% to 20 mass%, still more preferably from 0.3 mass% to 15 mass% with respect to the total amount of the oily composition from the viewpoint of improving the thickening effect of the oily composition to be obtained, and the touch feeling and use feeling of the cosmetic composition.

The silica particles (B) to be used in the present invention are not particularly limited as long as the particles are silica particles to be typically used in the field of, for example, a coating material, a pressure-sensitive adhesive or an adhesive, a fuel, a lubricant, food, or a cosmetic preparation, and various silica particles including silicic anhydride and silicon dioxide may be used. The shapes and forms of the silica particles to be used in the present invention are also not particularly limited, and for example, spherical silica particles, plate-shaped silica particles, porous silica particles, amorphous silica particles, fumed silica particles, non-crystalline silica particles, crystalline silica particles, colloidal silica, silica gel, silica aerogel, or silica sol may be used.

The particle diameters of the silica particles (B) to be used in the present invention are not particularly limited, and may be appropriately regulated in accordance with purposes, and for example, the average particle diameter thereof may be from 1 nm to 100 µm. The average particle diameter of the silica particles is preferably from 1 nm to 1 µm, more preferably from 1 nm to 500 nm, still more preferably from 1 nm to 100 nm from the viewpoint of improving the thickening effect of the oily composition to be obtained, and the touch feeling and use feeling of the cosmetic composition. In the present invention, the average particle diameter of the silica particles is measured by a laser diffraction method in conformity with JIS Z 8825 (2013).

The silica particles (B) to be used in the present invention may be hydrophilic silica particles or hydrophobic silica particles, but are preferably hydrophobic silica particles from the viewpoints of the effects of the present invention. For example, hydrophobic silica particles obtained by subjecting the surfaces of silica particles to hydrophobic treatment may be used as the hydrophobic silica particles, and a treatment method involving using any one of, for example, alkoxysilanes, silazanes, siloxanes, halogenated silanes, a saturated fatty acid, an amino acid, an ester, and a wax may be used as a method for the hydrophobic treatment. Hydrophobized silica particles subjected to dimethylsilylation treatment with any one of alkoxysilanes and halogenated silanes out of those materials are preferred.

A commercial product may be used as such silica particles, or the particles may be produced by a known method. Examples of commercial silica particles that may be used in the present invention include: an AEROSIL (trademark) series manufactured by Evonik Industries AG; an Efficium (trademark) series, a Tixosil (trademark) series, and a Zeosil (trademark) series manufactured by Solvay S.A.; a QSG series manufactured by Shin-Etsu Silicone; and a SEAHOSTAR (trademark) series manufactured by Nippon Shokubai Co., Ltd.

Although the content of the silica particles (B) in the oily composition of the present invention is not particularly limited, the content of the silica particles (B) in the oily composition of the present invention is preferably from 0.1 mass% to 25 mass%, more preferably from 0.2 mass% to 20 mass%, still more preferably from 0.3 mass% to 15 mass% with respect to the total amount of the oily composition from the viewpoint of the touch feeling, use feeling, and thickening effect of the oily composition to be obtained and the cosmetic composition.

In the present invention, when the above-mentioned silica particles are used as the silica particles (B), there can be obtained an oily composition, which imparts a satisfactory touch feeling and a satisfactory thickening effect to a product at the time of the combination of the silica particles with the oil component (C) through a synergistic thickening effect with the urethane polymer (A). In addition, in the present invention, combined use of the above-mentioned silica particles (B) with the urethane polymer (A) facilitates the maintenance of the transparent feeling of the oily composition to be obtained. Accordingly, when the particles and the polymer are used in a product, such as a coating material, a pressure-sensitive adhesive or an adhesive, a fuel, a lubricant, food, or a cosmetic preparation, various products having transparent appearances can be provided.

The oil component (C) to be used in the present invention is one or more kinds of oil components selected from an ester oil, a hydrocarbon oil, and a silicone oil. Examples of the ester oil that may be used in the present invention include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, glyceryl di-2-ethylhexanoate, a dipentaerythritol fatty acid ester, glyceryl monoisostearate, neopentyl glycol dicaprate, diisostearyl malate, glyceryl di-2-heptylundecanoate, trimethylolpropane tri-2-ethylhexanoate, trimethyolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, a castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, and 2-ethylhexyl succinate. Those ester oils may be used alone or in combination thereof.

The viscosity of the above-mentioned ester oil at 25°C is preferably from 1 mPa·s to 20,000 mPa·s, more preferably from 10 mPa·s to 10,000 mPa·s, still more preferably from 20 mPa·s to 8,000 mPa·s. In the present invention, the viscosity at 25°C is measured in conformity with JIS K 7117.

Examples of the hydrocarbon oil that may be used in the present invention include liquid paraffin, ozocerite, squalane, pristane, paraffin, ceresin, squalene, Vaseline, and microcrystalline wax. Those hydrocarbon oils may be used alone or in combination thereof.

The viscosity of the above-mentioned hydrocarbon oil at 25°C is preferably from 1 mPa·s to 200 mPa·s, more preferably from 5 mPa·s to 100 mPa·s, still more preferably from 10 mPa·s to 50 mPa·s.

Examples of the silicone oil that may be used in the present invention include: chain polysiloxanes (for example, dimethylpolysiloxane, methylphenylpolysiloxane, and diphenylpolysiloxane); cyclic polysiloxanes (for example, octamethylcyclotetrasiloxane, decamethylcyclopentasiloxane, and dodecamethylcyclohexasiloxane); silicone resins forming a three-dimensional network structure; silicone rubbers; and various modified polysiloxanes (an amino-modified polysiloxane, a polyether-modified polysiloxane, an alkyl-modified polysiloxane, a fluorine-modified polysiloxane, and the like). Those silicone oils may be used alone or in combination thereof.

The viscosity of the above-mentioned silicone oil at 25°C is preferably from 1 mPa·s to 200 mPa·s, more preferably from 5 mPa·s to 100 mPa·s, still more preferably from 10 mPa·s to 50 mPa·s.

The oil component (C) to be used in the present invention may be only one kind selected from the ester oil, the hydrocarbon oil, and the silicone oil, or may be a mixture of two or more kinds selected therefrom. It is preferable that the oil component (C) comprises the ester oil from the viewpoints of the effects of the present invention. In this case, the oil component (C) may consist of the ester oil, may be a mixture containing the ester oil and the hydrocarbon oil, or a mixture consisting of the ester oil and the hydrocarbon oil, may be a mixture containing the ester oil and the silicone oil, or a mixture consisting of the ester oil and the silicone oil, or may be a mixture containing the ester oil, the hydrocarbon oil, and the silicone oil, or a mixture consisting of the ester oil, the hydrocarbon oil, and the silicone oil.

Although the content of the oil component (C) in the oily composition of the present invention is not particularly limited, the content of the oil component (C) in the oily composition of the present invention is preferably from 5 mass% to 99 mass%, more preferably from 10 mass% to 99 mass%, still more preferably from 20 mass% to 98 mass% with respect to the total amount of the oily composition from the viewpoint of improving the thickening effect of the oily composition to be obtained, and the touch feeling and use feeling of the cosmetic composition.

In addition, in another embodiment, the content of the oil component (C) in the oily composition of the present invention is preferably from 50 mass% to 99 mass%, more preferably from 75 mass% to 99 mass%, still more preferably from 85 mass% to 98 mass%, particularly preferably from 90 mass% to 98 mass% with respect to the total amount of the oily composition.

Although a content ratio between the urethane polymer (A) and the silica particles (B) in the oily composition of the present invention is not particularly limited, the content ratio between the urethane polymer (A) and the silica particles (B) in the oily composition is preferably from 1:20 to 20:1, more preferably from 1:10 to 10:1, still more preferably from 1:5 to 5:1, particularly preferably from 1:5 to 3:1 in terms of mass ratio from the viewpoint of improving the thickening effect of the oily composition to be obtained, and the touch feeling and use feeling of the cosmetic composition.

Although a content ratio between the urethane polymer (A) and the oil component (C) in the oily composition of the present invention is not particularly limited, the content ratio between the urethane polymer (A) and the oil component (C) in the oily composition is preferably from 1:200 to 1:1, more preferably from 1:100 to 1:5, still more preferably from 1:50 to 1:10 in terms of mass ratio from the viewpoint of improving the thickening effect of the oily composition to be obtained, and the touch feeling and use feeling of the cosmetic composition.

Although a content ratio between the silica particles (B) and the oil component (C) in the oily composition of the present invention is not particularly limited, the content ratio between the silica particles (B) and the oil component (C) in the oily composition is preferably from 1:300 to 1:5, more preferably from 1:200 to 1:10, still more preferably from 1:100 to 1:15 in terms of mass ratio from the viewpoint of improving the thickening effect of the oily composition to be obtained, and the touch feeling and use feeling of the cosmetic composition.

The viscosity of the oily composition of the present invention is not particularly limited, and may be adjusted in accordance with purposes. However, for example, the viscosity of the oily composition of the present invention at 25°C is preferably from 100 mPa·s to 100,000 mPa·s, more preferably from 500 mPa·s to 80,000 mPa·s, still more preferably from 800 mPa·s to 50,000 mPa·s from the viewpoint that the viscosity of a product is easily adjusted, and hence a satisfactory touch feeling and a satisfactory thickening effect are imparted to the product. In the present invention, the viscosity at 25°C is measured in conformity with JIS K 7117.

Although a field in which the oily composition of the present invention may be used is not particularly limited, for example, the composition may be used in a product, such as a coating material, a pressure-sensitive adhesive or an adhesive, a fuel, a lubricant, food, or a cosmetic preparation. At this time, a known material may be incorporated into the product, such as a coating material, a pressure-sensitive adhesive or an adhesive, a fuel, a lubricant, food, or a cosmetic preparation, in accordance with its usage mode and purposes.

The cosmetic composition of the present invention is a cosmetic composition comprising the oily composition of the present invention. According to the present invention, there can be obtained a cosmetic composition whose viscosity, and touch feelings before and after its use are adjusted. Although the content of the oily composition in the cosmetic composition of the present invention is not particularly limited, for example, the oily composition of the present invention is preferably incorporated at from 0.01 mass% to 90 mass%, is more preferably incorporated at from 0.01 mass% to 75 mass%, and is still more preferably incorporated at from 0.01 mass% to 50 mass% with respect to the total amount of the cosmetic composition.

Although the content of the urethane polymer (A) in the cosmetic composition of the present invention is not particularly limited, the content of the urethane polymer (A) in the cosmetic composition of the present invention is preferably from 0.01 mass% to 20 mass%, more preferably from 0.1 mass% to 15 mass%, still more preferably from 0.2 mass% to 10 mass% with respect to the total amount of the cosmetic composition from the viewpoints of the use feeling and thickening effect of the cosmetic composition.

Although the content of the silica particles (B) in the cosmetic composition of the present invention is not particularly limited, the content of the silica particles (B) in the cosmetic composition of the present invention is preferably from 0.01 mass% to 20 mass%, more preferably from 0.1 mass% to 15 mass%, still more preferably from 0.2 mass% to 10 mass% with respect to the total amount of the cosmetic composition from the viewpoints of the use feeling and thickening effect of the cosmetic composition, and from the viewpoints of the use feeling and thickening effect of the silica particles (B) in the cosmetic composition.

Although the content of the oil component (C) in the cosmetic composition of the present invention is not particularly limited, the content of the oil component (C) in the cosmetic composition of the present invention is preferably from 1 mass% to 90 mass%, more preferably from 2 mass% to 90 mass%, still more preferably from 3 mass% to 80 mass% with respect to the total amount of the cosmetic composition from the viewpoints of the use feeling and thickening effect of the cosmetic composition.

The kind of a specific cosmetic preparation of the cosmetic composition of the present invention is not particularly limited, and includes, for example, a toiletry product, such as a shampoo or a rinse. Examples thereof include a toner, a cosmetic liquid, a milky lotion, a cream, a face-washing foam, a cleansing milk, a cleansing lotion, a hair tonic, a hair styling liquid, a setting lotion, a hair bleach, a color rinse, a permanent wave solution, a lipstick, a pack, a foundation, Eau de Cologne, a shampoo, a rinse, a treatment, a sunscreen, a deodorant, a perfume, a cleansing oil, and a cosmetic oil.

In addition to the oily composition described above, any other additive to be generally used in a cosmetic composition for improving and modifying various characteristics (e.g., solubility, dispersibility, stability, a use feeling, applicability, permeability, moisture retentivity, safety, a design property, an optical characteristic, aromaticity, and a whitening property) at the time of its storage, at the time of its use, and after the use in accordance with its use purposes may be used in the cosmetic composition of the present invention. Examples of the other additive include: a liquid oil and fat; a solid oil and fat; a wax; a powder component (provided that the urethane polymer (A) and silica particles (B) of the present invention are excluded); a higher fatty acid; an anionic surfactant; a cationic surfactant; an amphoteric surfactant; a non-ionic surfactant; a moisturizer; a polymer compound; a metal ion-sequestering agent; a lower alcohol; a polyhydric alcohol; a sugar; an amino acid and a derivative thereof; an organic amine; a pH-adjusting agent; an antioxidant; an antiseptic; a circulation promoter; an antiphlogistic agent; an activator; a whitening agent; an antiseborrheic agent; an anti-inflammatory agent; various extracts; and plant and seaweed extracts. Those additives may be arbitrarily blended alone or in combination thereof.

Examples of the liquid oil and fat include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, Japan tung oil, jojoba oil, germ oil, and triglycerin.

Examples of the solid oil and fat include cacao butter, coconut oil, hydrogenated coconut oil, palm oil, palm kernel oil, Japan wax kernel oil, hydrogenated oil, Japan wax, and hydrogenated castor oil.

Examples of the wax include beeswax, candelilla wax, cotton wax, carnauba wax, bayberry wax, privet wax, spermaceti, montan wax, bran wax, lanolin, kapok wax, lanolin acetate, liquid lanolin, sugar cane wax, a lanolin fatty acid isopropyl ester, hexyl laurate, reduced lanolin, jojoba wax, hard lanolin, shellac wax, a POE lanolin alcohol ether, a POE lanolin alcohol acetate, a POE cholesterol ether, a lanolin fatty acid polyethylene glycol, and a POE hydrogenated lanolin alcohol ether.

Examples of the powder component include: inorganic powders (for example, talc, kaolin, mica, sericite, white mica, bronze mica, synthetic mica, lepidolite, black mica, vermiculite, magnesium carbonate, calcium carbonate, aluminum silicate, barium silicate, calcium silicate, magnesium silicate, strontium silicate, a metal salt of tungstic acid, magnesium, zeolite, barium sulfate, calcined calcium sulfate (calcined gypsum), calcium phosphate, fluorine apatite, hydroxyapatite, ceramic powder, metal soap (for example, zinc myristate, calcium palmitate, or aluminum stearate), and boron nitride); organic powders (for example, polyamide resin powder (nylon powder), polyethylene powder, polymethyl methacrylate powder, polystyrene powder, copolymer resin powder of styrene and acrylic acid, benzoguanamine resin powder, polytetrafluoroethylene powder, and cellulose powder); inorganic white pigments (for example, titanium dioxide and zinc oxide); inorganic red pigments (for example, iron oxide (red iron oxide) and iron titanate); inorganic brown pigments (for example, γ-iron oxide); inorganic yellow pigments (for example, yellow iron oxide and yellow ocher); inorganic black pigments (for example, black iron oxide and lower titanium oxide); inorganic purple pigments (for example, manganese violet and cobalt violet); inorganic green pigments (for example, chromium oxide, chromium hydroxide, and cobalt titanate); inorganic blue pigments (for example, ultramarine blue and Prussian blue); pearl pigments (for example, titanium oxide-coated mica, titanium oxide-coated bismuth oxychloride, titanium oxide-coated talc, colored titanium oxide-coated mica, bismuth oxychloride, and argentine); metal powder pigments (for example, aluminum powder and copper powder); organic pigments, such as zirconium, barium, and aluminum lakes (for example, organic pigments, such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, and Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural coloring matter (for example, chlorophyl and β-carotene).

Examples of the higher fatty acid include lauric acid, myristic acid, palmitic acid, stearic acid, behenic acid, oleic acid, undecylenic acid, a tall oil fatty acid, isostearic acid, linoleic acid, linolenic acid, eicosapentaenic acid (EPA), and docosahexaenoic acid (DHA).

As a higher alcohol, there are given, for example: linear alcohols (for example, lauryl alcohol, cetyl alcohol, stearyl alcohol, behenyl alcohol, myristyl alcohol, oleyl alcohol, and cetostearyl alcohol); and branched alcohols (for example, 2-decyltetradecinol, lanolin alcohol, cholesterol, phytosterol, hexyl dodecanol, isostearyl alcohol, and octyl dodecanol).

Examples of the anionic surfactant include: fatty acid soaps (such as sodium laurate and sodium palmitate); higher alkyl sulfuric acid ester salts (such as sodium lauryl sulfate and potassium lauryl sulfate); alkyl ether sulfuric acid ester salts (such as POE-lauryl sulfate triethanolamine and POE-sodium lauryl sulfate); N-acylsarcosinates (such as sodium lauroylsarcosine); higher fatty acid amide sulfonic acid salts (such as N-myristoyl-N-methyl taurine sodium, coconut oil fatty acid methyl taurine sodium, and lauryl methyl taurine sodium); phosphoric acid ester salts (POE-sodium oleyl ether phosphate, POE-stearyl ether phosphoric acid, and the like); sulfosuccinic acid salts (such as sodium di-2-ethylhexyl sulfosuccinate, sodium monolauroyl monoethanolamide polyoxyethylene sulfosuccinate, and sodium lauryl polypropylene glycol sulfosuccinate); alkyl benzenesulfonic acid salts (such as sodium linear dodecylbenzenesulfonate, triethanolamine linear dodecylbenzenesulfonate, and linear dodecylbenzenesulfonic acid); higher fatty acid ester sulfuric acid ester salts (such as sodium hydrogenated coconut oil fatty acid glycerin sulfate); N-acyl glutamic acid salts (such as monosodium N-lauroyl glutamate, disodium N-stearoyl glutamate, and monosodium N-myristoyl-L-glutamate); sulfonated oils (such as Turkey red oil); POE-alkyl ether carboxylic acids; POE-alkyl allyl ether carboxylic acid salts; α-olefin sulfonic acid salts; higher fatty acid ester sulfonic acid salts; secondary alcohol sulfuric acid ester salts; higher fatty acid alkylolamide sulfuric acid ester salts; sodium lauroyl monoethanolamide succinate; N-palmitoyl aspartate ditriethanolamine; and casein sodium.

Examples of the cationic surfactant include: alkyltrimethylammonium salts (such as stearyltrimethylammonium chloride and lauryltrimethylammonium chloride); alkylpyridinium salts (such as cetylpyridinium chloride); dialkyldimethylammonium salts (such as distearyldimethylammonium chloride); alkyl quaternary ammonium salts; alkyldimethylbenzylammonium salts; alkylisoquinolinium salts; dialkylmorpholinium salts; POE-alkylamines; alkylamine salts; amyl alcohol fatty acid derivatives; benzalkonium chloride; and benzethonium chloride.

Examples of the amphoteric surfactant include: imidazoline-based amphoteric surfactants (such as sodium 2-undecyl-N,N,N-(hydroxyethylcarboxymethyl)-2-imidazoline, and 2-cocoyl-2-imidazolium hydroxide-1-carboxyethyloxy disodium salt); and betaine-based surfactants (such as a 2-alkyl-N-carboxymethyl-N-hydroxyethylimidazolium betaine (sodium cocoamphoacetate), betaine lauryldimethylaminoacetate, an alkylbetaine, amidobetaine, and sulfobetaine).

Examples of the non-ionic surfactant include: sorbitan fatty acid esters (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerin fatty acids (such as a monocottonseed oil fatty acid glycerin, glycerin monoerucate, glycerin sesquioleate, glycerin monostearate, glycerin α,α'-oleate pyroglutamate, and glycerin monostearate malate); propylene glycol fatty acid esters (such as propylene glycol monostearate); hydrogenated castor oil derivatives; glycerin alkyl ethers; POE-sorbitan fatty acid esters (such as POE-sorbitan monooleate, POE-sorbitan monostearate, and POE-sorbitan tetraoleate); POE-sorbit fatty acid esters (such as POE-sorbit monolaurate, POE-sorbit monooleate, POE-sorbit pentaoleate, and POE-sorbit monostearate); POE-glycerin fatty acid esters (such as POE-glycerin monostearate, POE-glycerin monoisostearate, and POE-glycerin triisostearate); POE-fatty acid esters (such as POE-distearate, POE-monodioleate, and ethylene glycol distearate); POE-alkyl ethers (such as POE-lauryl ether, POE-oleyl ether, POE-stearyl ether, POE-behenyl ether, POE-2-octyldodecyl ether, and POE-cholestanol ether); Pluronic-type surfactants (such as Pluronic); POE/POP-alkyl ethers (such as POE/POP-cetyl ether, POE/POP-2-decyl tetradecyl ether, POE/POP-monobutyl ether, POE/POP-hydrogenated lanolin, and POE/POP-glycerin ether); tetra POE/tetra POP-ethylenediamine condensates (such as Tetronic); POE-castor oil/hydrogenated castor oil derivatives (such as POE-castor oil, POE-hydrogenated castor oil, POE-hydrogenated castor oil monoisostearate, POE-hydrogenated castor oil triisostearate, POE-hydrogenated castor oil monopyroglutamic acid monoisostearic acid diester, and POE-hydrogenated castor oil maleic acid ester); POE-beeswax/lanolin derivatives (such as POE-sorbit beeswax); alkanolamides (such as coconut oil fatty acid diethanolamides, lauric acid monoethanol amide, and a fatty acid isopropanolamide); POE-propylene glycol fatty acid esters; POE-alkylamines; POE-fatty acid amides; sucrose fatty acid esters; alkylethoxydimethylamine oxides; and trioleyl phosphate.

Examples of the moisturizer include polyethylene glycol, xylitol, sorbitol, maltitol, chondroitin sulfate, hyaluronic acid, mucoitin sulfate, charonic acid, atelocollagen, cholesteryl-12-hydroxystearate, sodium lactate, a bile acid salt, a dl-pyrrolidonecarboxylic acid salt, short-chain soluble collagen, a diglycerin (EO)PO adduct, a *Rosa roxburghii* extract, a yarrow extract, and a sweet clover extract.

As a natural water-soluble polymer, there are given, for example: plant-based polymers (such as gum arabic, tragacanth gum, galactan, guar gum, carob gum, gum karaya, carrageenan, pectin, agar, a quince seed (quince), algae colloid (brown alga extract), starch (rice, corn, potato, and wheat), and glycyrrhizic acid); microorganism-based polymers (such as xanthane gum, dextran, succinoglucan, pullulan, and gellan gum); and animal-based polymers (such as collagen, casein, albumin, and gelatin).

Examples of the water-soluble polymer include: starch-based polymers (such as carboxymethyl starch and methylhydroxypropyl starch); cellulose-based polymers (such as methylcellulose, ethylcellulose, methylhydroxypropylcellulose, hydroxyethylcellulose, sodium cellulose sulfate, hydroxypropylcellulose, carboxymethylcellulose, sodium carboxymethylcellulose, crystalline cellulose, and cellulose powder); alginic acid-based polymers (such as sodium alginate and propylene glycol alginate ester); vinyl-based polymers (such as polyvinyl alcohol, polyvinyl methyl ether, polyvinylpyrrolidone, and a carboxyvinyl polymer); polyoxyethylene-based polymers (such as polyoxyethylene-polyoxypropylene copolymers of polyethylene glycol 20,000, polyethylene glycol 40,000, or polyethylene glycol 60,000); acrylic polymers (such as sodium polyacrylate, polyethyl acrylate, and polyacrylamide); polyethylene imine; and cationic polymers.

As a chelating agent, there are given, for example, 1-hydroxyethane-1,1-diphosphonic acid, 1-hydroxyethane-1,1-diphosphonic acid tetrasodium salts, disodium edetate, trisodium edetate, tetrasodium edetate, sodium polyphosphate, sodium metaphosphate, phosphoric acid, ascorbic acid, succinic acid, and edetic acid.

Examples of the lower alcohol include ethanol, propanol, isopropanol, isobutyl alcohol, and t-butyl alcohol.

Examples of the polyhydric alcohol include: dihydric alcohols (such as 1,2-ethanediol, 1,2-propanediol, 1,3-propanediol, 1,2-butanediol, 1,3-butanediol, 1,4-butanediol, 2,3-butanediol, 1,2-pentanediol, 1,5-pentanediol, and 2,4-pentanediol); trihydric alcohols (such as glycerin and trimethylolpropane); tetrahydric alcohols (such as 1,2,5,6-hexanetetraol and pentaerythritol); pentahydric alcohols (such as xylitol); hexahydric alcohols (such as sorbitol and mannitol); polyhydric alcohol polymers (such as diethylene glycol, triethylene glycol, dipropylene glycol, polypropylene glycol, tetraethylene glycol, diglycerin, polyethylene glycol, triglycerin, tetraglycerin, and polyglycerin); alkyl glyceryl ethers (such as propyl glyceryl ether, butyl glyceryl ether, pentyl glyceryl ether, hexyl glyceryl ether, cyclohexyl glyceryl ether, heptyl glyceryl ether, octyl glyceryl ether, ethylhexyl glyceryl ether, nonyl glyceryl ether, decyl glyceryl ether, hexadecyl glyceryl ether, oleyl glyceryl ether, octadecyl glyceryl ether, and isostearyl glyceryl ether); alcohol alkyl ethers (such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); sugar alcohols (such as sorbitol, maltitol, maltotriose, mannitol, erythritol, glucose, fructose, amylolysis sugars, maltose, xylitose, and alcohols prepared by reduction of amylolysis sugars); Glysolid; tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin ether phosphate; and POP/POE-pentaneerythritol ether.

As a monosaccharide, there are given, for example: trioses (such as D-glyceryl aldehyde and dihydroxyacetone); tetroses (such as D-erythrose, D-erythrulose, D-threose, and erythritol); pentoses (such as L-arabinose, D-xylose, L-lixose, D-arabinose, D-ribose, D-ribulose, D-xylulose, and L-xylulose); hexoses (such as D-glucose, D-talose, D-psicose, D-galactose, D-fructose, L-galactose, L-mannose, and D-tagatose); heptoses (such as aldoheptose and heptulose); octoses (such as octulose); deoxy sugars (such as 2-deoxy-D-ribose, 6-deoxy-L-galactose, and 6-deoxy-L-mannose); amino sugars (such as D-glucosamine, D-galactosamine, sialic acid, aminouronic acid, and muramic acid); and uronic acids (such as D-glucuronic acid, D-mannuronic acid, L-guluronic acid, D-galacturonic acid, and L-iduronic acid).

As an oligosaccharide, there are given, for example, sucrose, umbelliferose, lactose, planteose, isolychnoses, α,α-trehalose, raffinose, lychnoses, umbilicin, stachyose, and verbascoses.

As a polysaccharide, there are given, for example, cellulose, quince seed, chondroitin sulfate, starch, galactan, dermatan sulfate, glycogen, gum arabic, heparan sulfate, hyaluronic acid, tragacanth gum, keratan sulfate, chondroitin, xanthane gum, mucoitin sulfate, guar gum, dextran, keratosulfate, locust bean gum, succinoglucan, and charonic acid.

Examples of the amino acid include: neutral amino acids (such as threonine and cysteine); and basic amino acids (such as hydroxylysine). In addition, as an amino acid derivative, there are given, for example, acylsarcosine sodium (lauroylsarcosine sodium), acylglutamic acid salts, acyl β-alanine sodium, glutathione, and pyrrolidone carboxylic acid.

Examples of the organic amine include monoethanol amine, diethanol amine, triethanol amine, morpholine, triisopropanolamine, 2-amino-2-methyl-1,3-propanediol, and 2-amino-2-methyl-1-propanol.

As a polymer emulsion, there are given, for example, an acrylic resin emulsion, a polyethyl acrylate emulsion, an acrylic resin liquid, a polyacrylic alkyl ester emulsion, a polyvinyl acetate resin emulsion, and a natural rubber latex.

Examples of the pH-adjusting agent include lactic acid-sodium lactate, succinic acid-sodium succinate, citric acid-sodium citrate, and sodium hydrogen carbonate. It is only required that the pH of the cosmetic composition of the present invention be appropriately regulated in accordance with applications. The pH is preferably from 2.0 to 12.0 from the viewpoint of applicability to the skin or the like.

As a vitamin, there are given, for example, vitamins A, B1, B2, B6, C, and E, and derivatives thereof, pantothenic acid and a derivative thereof, and biotin.

Examples of the antioxidant include tocopherols, dibutylhydroxytoluene, butylhydroxyanisole, and gallic acid esters.

Examples of other blendable components include: antiphlogistic agents (such as glycyrrhizic acid derivatives, glycyrrhetinic acid derivatives, salicylic acid derivatives, hinokitiol, zinc oxide, and allantoin); whitening agents (such as a saxifrage extract and arbutin); various extracts (extracts of *Phellodendron bark, Coptis japonica,* lithospermum root, *Paeonia lactiflora, Swertia japonica,* birch, sage, loquat, carrot, aloe, mallow, iris, grapevine, coix seed, dishcloth gourd, lily, saffron, *Cnidium rhizome,* ginger, hypericum, *Ononis spinosa,* garlic, capsicum, *Citrus unshiu* peel, *Angelica acutiloba,* seaweed, or the like); activators (such as royal jelly, a photosensitizer, and cholesterol derivatives); circulation promoters (such as benzyl nicotinate ester, β-butoxyethyl nicotinate ester, capsaicin, zingerone, Cantharides tincture, ichthammol, tannic acid, α-borneol, tocopherol nicotinate, inositol hexanicotinate, cyclandelate, cinnarizine, tolazoline, acetylcholine, verapamil, cepharanthine, and γ-orizanol); antiseborrheic agents (such as sulfur and thianthol); and anti-inflammatory agents (such as tranexamic acid, thiotaurine, and hypotaurine).

The viscosity of the cosmetic composition of the present invention is not particularly limited, and may be adjusted in accordance with the kind of a specific cosmetic preparation thereof and purposes. However, for example, the viscosity of the cosmetic composition of the present invention at 25°C is preferably from 100 mPa·s to 100,000 mPa·s, more preferably from 200 mPa·s to 80,000 mPa·s, still more preferably from 300 mPa·s to 50,000 mPa·s from the viewpoint of the use feeling of the cosmetic composition. In the present invention, the viscosity at 25°C is measured in conformity with JIS K 7117.

### Examples

Details about the present invention are described below by way of the Examples and the Comparative Examples, but the present invention is not limited by these examples. In Examples and the like below, the term "%" is on a mass basis unless otherwise stated.

### [Production of (PPG-12/SMDI) Copolymer 1]

872.7 Grams (1.25 mol) of PPG-12 and 135.15 g (0.40 mol) of dicyclohexylmethane 4,4'-diisocyanate were loaded into a glass-made reaction vessel with a stirring machine, a cooling tube, and a nitrogen-introducing tube, and were caused to react with each other at 120°C for 1 hour. The fact that no NCO absorption was present was recognized by the infrared absorption spectrum of the reaction product, and then the rection was terminated. Thus, a (PPG-12/SMDI) copolymer 1 was produced. The weight-average molecular weight of the resultant (PPG-12/SMDI) copolymer 1 determined in terms of styrene through measurement by gel permeation chromatography (GPC) was 4,600.

### [Production of (Polyglyceryl-2 Diisostearate/IPDI) Copolymer 1]

920.9 Grams (1.27 mol) of polyglyceryl-2 diisostearate and 112.14 g (0.40 mol) of isophorone diisocyanate were loaded into a glass-made reaction vessel with a stirring machine, a cooling tube, and a nitrogen-introducing tube, and were caused to react with each other at 120°C for 1 hour. The fact that no NCO absorption was present was recognized by the infrared absorption spectrum of the reaction product, and then the rection was terminated. Thus, a (polyglyceryl-2 diisostearate/IPDI) copolymer 1 was produced. The weight-average molecular weight of the resultant (polyglyceryl-2 diisostearate/IPDI) copolymer 1 determined in terms of styrene through measurement by gel permeation chromatography (GPC) was 2,900.

### [Production of (PPG-51/SMDI) Copolymer 1 (Comparative Compound)]

981.3 Grams (0.33 mol) of PPG-51 and 38.7 g (0.15 mol) of dicyclohexylmethane 4,4'-diisocyanate were loaded into a glass-made reaction vessel with a stirring machine, a cooling tube, and a nitrogen-introducing tube, and were caused to react with each other at 120°C for 1 hour. The fact that no NCO absorption was present was recognized by the infrared absorption spectrum of the reaction product, and then the rection was terminated. Thus, a (PPG-51/SMDI) copolymer 1 was produced. The weight-average molecular weight of the resultant (PPG-51/SMDI) copolymer 1 determined in terms of styrene through measurement by gel permeation chromatography (GPC) was 9,200.

### [Oily Composition Preparation 1]

The following urethane polymers, silica particles, and oil components were used, and the respective materials were mixed as shown in Tables 1 to 3 below to prepare each of oily compositions of Examples 1 to 5 and Comparative Examples 1 to 9. Each of the oily compositions of Examples 1 to 5 and Comparative Examples 1 to 9 thus prepared had high transparency. In Tables 1 to 3, the term "%" represents "mass%".

### <Urethane Polymer (A)>

Urethane polymer 1: (PPG-12/SMDI) copolymer 1
Urethane polymer 2: (Polyglyceryl-2 diisostearate/IPDI) copolymer 1
Urethane polymer 3 (comparative compound):
   (PPG-51/SMDI) copolymer 1

### <Silica Particles (B)>

Silica particles 1: Hydrophobic silica particles that are subjected to dimethylsilylation treatment and have an average primary particle diameter of 16 nm (manufactured by Evonik Industries AG, AEROSIL (trademark) R972)

### <Oil Component (C)>

Ester oil 1: Diisostearyl malate (viscosity at 25°C: 5,500 mPa·s)
Ester oil 2: Trioctanoin (viscosity at 25°C: 40 mPa·s)

**Table 1**

| | | Example 1 | Example 2 | Example 3 | Example 4 | Example 5 |
|---|---|---|---|---|---|---|
| Uretha ne polyme r | Urethane polymer 1 | 1.0% | 2.5% | 2.0% | | |
| | Urethane polymer 2 | | | | 1.0% | 2.0% |
| | Urethane polymer 3 | | | | | |
| Silica partic les | Silica particles 1 | 4.0% | 2.5% | 5.0% | 4.0% | 5.0% |
| Oil compon ent | Ester oil 1 | 50% | 50% | 48.9% | 50% | 48.9% |
| | Ester oil 2 | 45% | 45% | 44.1% | 45% | 44.1% |
| Viscosity (mPa·s) | | 5,000 | 1,000 | 5,500 | 2,000 | 17,000 |
| Result of touch feeling evaluation | | ○ | ○ | ○ | ○ | ○ |

**Table 2**

| | | Comparative Example 1 | Comparative Example 2 | Comparative Example 3 | Comparative Example 4 | Comparative Example 5 |
|---|---|---|---|---|---|---|
| Urethane polymer | Urethane polymer 1 | 5.0% | | | | |
| | Urethane polymer 2 | | 5.0% | | | |
| | Urethane polymer 3 | | | 5.0% | | 1.0% |
| Silica particles | Silica particles 1 | | | | 5.0% | 4.0% |
| Oil component | Ester oil 1 | 50% | 50% | 50% | 50% | 50% |
| | Ester oil 2 | 45% | 45% | 45% | 45% | 45% |
| Viscosity (mPa·s) | | 300 | 300 | 300 | 1,000 | 400 |
| Result of touch feeling evaluation | | ○ | ○ | ○ | × | ○ |

**Table 3**

| | | Comparative Example 6 | Comparative Example 7 | Comparative Example 8 | Comparative Example 9 |
|---|---|---|---|---|---|
| Urethane polymer | Urethane polymer 1 | | | | |
| | Urethane polymer 2 | | | | |
| | Urethane polymer 3 | 2.5% | 5.0% | 5.0% | |
| Silica particles | Silica particles 1 | 2.5% | 2.5% | 5.0% | |
| Oil component | Ester oil 1 | 50% | 48.7% | 47.4% | 52.6% |
| | Ester oil 2 | 45% | 43.8% | 42.6% | 47.4% |
| Viscosity (mPa·s) | | 300 | 450 | 1,000 | 250 |
| Result of touch feeling evaluation | | ○ | ○ | × | ○ |

### <Viscosity Measurement>

Each of the oily compositions prepared in Examples 1 to 5 and Comparative Examples 1 to 9 was held in a thermostatic bath at 25°C for 1 hour, and then its viscosity was measured in conformity with JIS K 7117 with a B-type viscometer at 25°C and a number of revolutions of 5 rpm. The results of the measurement are shown in Tables 1 to 3.

### <Touch Feeling Evaluation>

A touch feeling at the time of the application of an appropriate amount of each of the oily compositions prepared in Examples 1 to 5 and Comparative Examples 1 to 9 to the back of a hand, and that after the application were evaluated by the following evaluation criteria. The results of the evaluation are shown in Tables 1 to 3.
Criteria for touch feeling evaluation
∘: A sticky feeling is absent, or is substantially unfelt.
×: A sticky feeling is clearly present.

### [Oily Composition Preparation 2]

The following urethane polymers, silica particles, and oil components were used, and the respective materials were mixed as shown in Tables 4 and 5 below to prepare each of oily compositions of Examples 6 to 12 and Comparative Examples 10 to 15. Each of the oily compositions of Examples 6 to 12 and Comparative Examples 10 to 15 thus prepared had high transparency. In Tables 4 and 5, the term "%" represents "mass%".

### <Urethane Polymer (A)>

Urethane polymer 1: (PPG-12/SMDI) copolymer 1

### <Silica Particles (B)>

Silica particles 1: Hydrophobic silica particles that are subjected to dimethylsilylation treatment and have an average primary particle diameter of 16 nm (manufactured by Evonik Industries AG, AEROSIL (trademark) R972)

### <Oil Component (C)>

Ester oil 1: Diisostearyl malate (viscosity at 25°C: 5,500 mPa·s)
Ester oil 2: Trioctanoin (viscosity at 25°C: 40 mPa·s)
Silicone oil 1: Diphenylsiloxy phenyl trimethicone (viscosity at 25°C: 15 mPa·s)
Hydrocarbon oil 1: Liquid paraffin (viscosity at 25°C: 20 mPa·s)

### <Viscosity Measurement>

Each of the oily compositions prepared in Examples 6 to 12 and Comparative Examples 10 to 15 was held in a thermostatic bath at 25°C for 1 hour, and then its viscosity was measured in conformity with JIS K 7117 with a B-type viscometer at 25°C and a number of revolutions of 5 rpm. The results of the measurement are shown in Tables 4 and 5.

### <Touch Feeling Evaluation>

A touch feeling at the time of the application of an appropriate amount of each of the oily compositions prepared in Examples 6 to 12 and Comparative Examples 10 to 15 to the back of a hand, and that after the application were evaluated by the following evaluation criteria. The results of the evaluation are shown in Tables 4 and 5.
Criteria for touch feeling evaluation
∘: A sticky feeling is absent, or is substantially unfelt.
×: A sticky feeling is clearly present.

**Table 4**

| | | Example 6 | Example 7 | Example 8 | Example 9 | Comparative Example 10 | Comparative Example 11 | Comparative Example 12 |
|---|---|---|---|---|---|---|---|---|
| Urethane polymer | Urethane polymer 1 | 5.0% | 1.0% | 2.0% | 5.0% | | 5.0% | |
| | Urethane polymer 2 | | | | | | | |
| | Urethane polymer 3 | | | | | | | |
| Silica particles | Silica particles 1 | 5.0% | 4.0% | 5.0% | 5.0% | | | 5.0% |
| Oil component | Ester oil 1 | 50.0% | 50.0% | 50.0% | 50.0% | 50.0% | 50.0% | 50.0% |
| | Ester oil 2 | 40.0% | | | | | | |
| | Silicone oil 1 | | 45.0% | 43.0% | 40.0% | 50.0% | 45.0% | 45.0% |
| | Hydrocarb on oil 1 | | | | | | | |
| Viscosity (mPa·s) | | 5,700 | 26,300 | 26,700 | 27,500 | 15 | 30 | 310 |
| Result of touch feeling evaluation | | ○ | ○ | ○ | ○ | ○ | ○ | × |

**Table 5**

| | | Example 10 | Example 11 | Example 12 | Comparative Example 13 | Comparative Example 14 | Comparative Example 15 |
|---|---|---|---|---|---|---|---|
| Urethane polymer | Urethane polymer 1 | 1.0% | 2.0% | 5.0% | | 5.0% | |
| | Urethane polymer 2 | | | | | | |
| | Urethane polymer 3 | | | | | | |
| Silica particles | Silica particles 1 | 4.0% | 5.0% | 5.0% | | | 5.0% |
| Oil component | Ester oil 1 | 50.0% | 50.0% | 50.0% | 50.0% | 50.0% | 50.0% |
| | Ester oil 2 | | | | | | |
| | Silicone oil 1 | | | | | | |
| | Hydrocarb on oil 1 | 45.0% | 43.0% | 40.0% | 50.0% | 45.0% | 45.0% |
| Viscosity (mPa·s) | | 12,800 | 13,200 | 16,700 | 20 | 50 | 1,200 |
| Result of touch feeling evaluation | | ○ | ○ | ○ | ○ | ○ | × |

It is understood from the above-mentioned results that the oily composition of the present invention uses a specific urethane polymer and the silica particles in combination, and hence shows a satisfactory touch feeling and a significant thickening effect as compared to those in the cases where only the urethane polymers are blended (Comparative Examples 1 and 2, 11, and 14), and the cases where only the silica particles are blended (Comparative Examples 4, 12, and 15). In addition, the effect is an effect that is not observed in the cases where the other urethane polymer and the silica particles are used (Comparative Examples 5 to 8). Accordingly, the fact that the adoption of the configuration provides an oily composition showing a satisfactory touch feeling and a satisfactory thickening effect is a significant effect that cannot be expected by a person skilled in the art, and hence the present invention is an invention extremely useful in the field of, for example, a coating material, a pressure-sensitive adhesive or an adhesive, a fuel, a lubricant, food, or a cosmetic preparation.

## Claims

1. An oily composition, comprising:
at least one kind of urethane polymer (A) selected from the group consisting of a (PPG-12/SMDI) copolymer and a (polyglyceryl-2 diisostearate/IPDI) copolymer;
silica particles (B); and
one or more kinds of oil components (C) selected from an ester oil, a hydrocarbon oil, and a silicone oil.

2. The oily composition according to claim 1, wherein a content ratio between the urethane polymer (A) and the silica particles (B) is from 1:20 to 20:1 in terms of mass ratio.

3. The oily composition according to claim 1 or 2, wherein the oil component (C) contains the ester oil.

4. The oily composition according to any one of claims 1 to 3, wherein a content ratio between the urethane polymer (A) and the oil component (C) is from 1:200 to 1:1 in terms of mass ratio.

5. The oily composition according to any one of claims 1 to 4, wherein the silica particles (B) have an average particle diameter of from 1 nm to 1 µm.

6. The oily composition according to any one of claims 1 to 5, wherein the silica particles (B) are hydrophobic silica particles.

7. The oily composition according to any one of claims 1 to 6, wherein the oily composition is used in preparation of a cosmetic preparation.

8. A cosmetic composition, comprising the oily composition of any one of claims 1 to 7.

9. The cosmetic composition according to claim 8, wherein a content of the urethane polymer (A) is from 0.01 mass% to 10.0 mass% with respect to a total amount of the cosmetic composition.

10. A use of the oily composition of any one of claims 1 to 6 for preparing a cosmetic composition.

11. A method of thickening one or more kinds of oil components (C) selected from an ester oil, a hydrocarbon oil, and a silicone oil, the method comprising blending the oil components (C) with at least one kind of urethane polymer (A) selected from the group consisting of a (PPG-12/SMDI) copolymer and a (polyglyceryl-2 diisostearate/IPDI) copolymer, and silica particles (B).
